# EUROPÄISCHE PATENTANMELDUNG

(11) **EP 4 628 281 A1**
(43) Veröffentlichungstag der Anmeldung: **08.10.2025**
(21) Anmeldenummer: 25152580.4
(22) Anmeldetag: 17.01.2025
(51) Int. Cl.: B29C 49/42, B29C 49/02, B29C 49/64, B29C 49/46

(54) **ANLAGE UND VERFAHREN ZUM HERSTELLEN UND BEHANDELN VON BEHÄLTERN**

(30) Priorität: 03.04.2024 DE 102024109306
(71) Anmelder: Krones AG, 93073 Neutraubling (DE)
(72) Erfinder: URBAN, Ralf, 93073 Neutraubling (DE); HIRDINA, Jochen, 93073 Neutraubling (DE)
(74) Vertreter: Grünecker Patent- und Rechtsanwälte PartG mbB

(57) **Zusammenfassung**

Anlage zum Herstellen und Behandeln von Behältern, die Anlage umfassend einen Reinraum, eine Herstellungsmaschine zum Herstellen von Vorformlingen, eine stromab der Herstellungsmaschine angeordnete Blasformmaschine zum Ausformen der Vorformlinge zu Behältern und eine stromab der Blasformmaschine angeordnete Behälterbehandlungsmaschine zum Behandeln der Behälter, wobei die Herstellungsmaschine, die Blasformmaschine und die Behälterbehandlungsmaschine im Reinraum angeordnet sind und wobei jede mit einem Vorformling oder einem Behälter in physischen Kontakt kommende Komponente der Herstellungsmaschine, der Blasformmaschine und der Behälterbehandlungsmaschine aus sterilisationsbeständigem Material besteht oder sterilisationsbeständiges Material umfasst.

## Beschreibung

Die vorliegende Erfindung betrifft eine Anlage zum Herstellen und Behandeln von Behältern gemäß Anspruch 1 sowie ein Verfahren zum Herstellen und Behandeln von Behältern mit einer Anlage gemäß Anspruch 12.

### Stand der Technik

Anlagen zum Herstellen und/oder Behandeln von Behältern sind aus dem Stand der Technik hinreichend bekannt.

So offenbart beispielsweise die DE 10 2011 056 293 A1 eine Anlage, umfassend eine Spritzgussmaschine und nachgeordnete Einrichtungen, insbesondere eine Umformungseinrichtung und eine Etikettiermaschine, die in einem Reinraum angeordnet sind und so die Sterilität der in der Spritzgussmaschine hergestellten Vorformlinge sicherstellen.

Die EP 4 122 872 A1 offenbart weiterhin eine Anlage in einem Schutzgehäuse, so dass bestimmte Reinraumanforderungen gewährleistet werden können.

Die DE 10 2015 105 994 A1 beschreibt eine verblockte Anlage, in der eine Spritzgussmaschine, eine Blasformmaschine sowie eine Fülleinrichtung innerhalb eines Reinraums angeordnet sind und so möglichst sterile Bedingungen realisiert werden können.

Die bekannten Anlagen erlauben zwar grundsätzlich das Herstellen und weitere Behandeln von Behältern unter Reinraumbedingungen. Jedoch kann ein Eintrag von nicht sterilen Komponenten oder Materialien bei nachfolgenden Prozessen zu Verkeimungen der Behälter führen, was sich negativ auf die Qualität der Behälter und insbesondere Produkte auswirken kann.

### Aufgabe

Ausgehend vom bekannten Stand der Technik besteht die zu lösende technische Aufgabe somit darin, eine Anlage zum Herstellen und Behandeln von Behältern sowie ein Verfahren zum Herstellen und Behandeln von Behältern mit einer Anlage anzugeben, mit denen auch der nachträgliche Eintrag von Verunreinigungen bei möglichst geringem Wartungsaufwand behandelt werden kann.

### Lösung

Diese Aufgabe durch die Anlage zum Herstellen und Behandeln von Behältern gemäß Anspruch 1 sowie das Verfahren zum Herstellen und Behandeln von Behältern mit einer Anlage gemäß Anspruch 12 gelöst. Vorteilhafte Weiterbildungen der Erfindung sind in den Unteransprüchen erfasst.

Die erfindungsgemäße Anlage zum Herstellen und Behandeln von Behältern umfasst einen Reinraum, eine Herstellungsmaschine zum Herstellen von Vorformlingen, eine stromab der Herstellungsmaschine angeordnete Blasformmaschine zum Ausformen der Vorformlinge zu Behältern und eine stromab der Blasformmaschine angeordnete Behälterbehandlungsmaschine zum Behandeln der Behälter, wobei die Herstellungsmaschine, die Blasformmaschine und die Behälterbehandlungsmaschine im Reinraum angeordnet sind und wobei jede mit einem Vorformling oder einem Behälter in physischen Kontakt kommende Komponente der Herstellungsmaschine, der Blasformmaschine und der Behälterbehandlungsmaschine aus sterilisationsbeständigem Material besteht oder sterilisationsbeständiges Material umfasst.

Unter Behältern sind in diesem Zusammenhang insbesondere Flaschen oder Dosen oder Tuben oder ähnliche Objekte zu verstehen, die aus Vorformlingen hergestellt werden können.

Unter den mit einem Vorformling oder einem Behälter in physischen Kontakt kommenden Komponenten der Herstellungsmaschine, der Blasformmaschine und der Behälterbehandlungsmaschine sind solche Komponenten bzw. Bestandteile dieser Maschinen zu verstehen, die in direkten physischen Kontakt mit den Vorformlingen oder Behälter kommen, also diesen unmittelbar ohne weitere Materialien dazwischen an seiner inneren oder äußeren Oberfläche berühren. Das sterilisationsbeständige Material ist insbesondere als ein solches Material zu verstehen, das bei Beaufschlagen dieser Komponenten mit einem Sterilisationsmedium nicht korrodiert oder eine sonstige Qualitätsverschlechterung bzw. Materialdegeneration erfährt.

Mit dieser Anlage ist es möglich, neben der grundsätzlich gewährleisteten Sterilität der Atmosphäre im Reinraum auch zusätzliche Sterilisationsschritte vorzunehmen, sofern es beispielsweise unabsichtlich oder absichtlich zu Verunreinigungen der Atmosphäre und/oder der Komponenten kommt, die sich nachteilig auf die Qualität der hergestellten Behälter auswirken könnten. Durch die Verwendung von sterilisationsbeständigem Material wird hierbei ein notwendiger Austausch von Komponenten aufgrund von Verschleiß vermieden.

In einer Ausführungsform ist vorgesehen, dass das sterilisationsbeständige Material beständig gegenüber Beaufschlagung mit einem gasförmigen und/oder flüssigen Sterilisationsmedium ist. Das gasförmige oder flüssige Sterilisationsmedium hat bevorzugt antibakterielle und/oder antivirale und/oder fungizide Eigenschaften. Hiermit wird eine zuverlässige Sterilisation der Komponenten ermöglicht und gleichzeitig Verschleiß dieser auch bei häufiger Sterilisation vermieden.

Insbesondere kann vorgesehen sein, dass das Sterilisationsmedium H₂O₂ und/oder O₃ und/oder Peressigsäure ist. Diese Sterilisationsmedien kommen üblicherweise in der getränkeverarbeitenden Industrie oder allgemein der Lebensmittel- sowie Kosmetikindustrie und Medizintechnik zum Einsatz. Hiergegen beständige Materialien können daher besonders vorteilhaft verwendet werden.

Es kann vorgesehen sein, dass die Anlage eine Reinraumsterilisationseinrichtung umfasst, die zumindest einen Bereich des Reinraums mit einem Sterilisationsmedium beaufschlagen kann. Die Reinraumsterilisationseinrichtung kann insbesondere so ausgebildet sein, dass sie den gesamten Reinraum mit einem Sterilisationsmedium (beispielsweise H₂O₂ oder Ozon oder Peressigsäure) beaufschlagen kann, um beispielsweise nach Umrüstarbeiten der Anlage die Reinraumqualität wiederherzustellen. Dabei kann das Beaufschlagen mit Sterilisationsmedium bevorzugt während einer Reinigungsphase oder eines Reinigungsbetriebs erfolgen, wobei der Reinigungsbetrieb eine Betriebsart der Anlage ist, während der keine Behälter hergestellt und/oder behandelt werden. Hierdurch wird vorteilhaft vermieden, dass hergestellte und/oder behandelte Behälter durch das Sterilisationsmedium beeinflusst werden.

Es kann vorgesehen sein, dass die Anlage eine Vorformling-Sterilisationseinrichtung umfasst, die Vorformlinge mit einem Sterilisationsmedium beaufschlagen kann.

Sofern im Herstellungsprozess der Vorformlinge ein Risiko für Kontamination der Vorformlinge oder nicht ausreichender Sterilisation des Vorformlingsmaterials besteht (beispielsweise aufgrund zu niedriger Herstellungstemperatur der Vorformlinge), kann die Vorformling-Sterilisationseinrichtung vorteilhaft genutzt werden, um die Sterilität der Vorformlinge vor dem Ausformen zu Behältern sicherzustellen, wobei durch die Verwendung sterilisationsbeständiger Materialien für die Komponenten (hier insbesondere Greifer oder Klammern zum Befördern der Vorformlinge) der Verschleiß vorteilhaft minimiert wird.

Insbesondere kann vorgesehen sein, dass die Anlage eine Steuereinheit umfasst, die ausgebildet ist, die Vorformling-Sterilisationseinrichtung abhängig von einem für eine Sterilität der Vorformlinge indikativen Parameter zum Beaufschlagen der Vorformlinge mit dem Sterilisationsmedium zu steuern.

Die Steuereinheit kann als Computer mit zugeordnetem Speicher und/oder Prozessor ausgeführt sein, der beispielsweise durch Vergleich eines gemessenen indikativen Parameters einer oder mehrerer Vorformlinge mit einem Grenzwert, dessen Überschreiten oder Unterschreiten durch den gemessenen indikativen Parameter ein Kontaminationsrisiko anzeigt, die Vorformling-Sterilisationseinrichtung steuert, um durch Ausbringen von Sterilisationsmedium das Kontaminationsrisiko zu reduzieren. Dies kann bevorzugt so ausgeführt sein, dass die Vorformling-Sterilisationseinrichtung nur dann zum Beaufschlagen der Vorformlinge mit dem Sterilisationsmedium genutzt wird, wenn ein Kontaminationsrisiko besteht, also der indikative Parameter indikativ für ein solches Kontaminationsrisiko ist (den Grenzwert also entsprechend überschreitet oder unterschreitet).

Es kann vorgesehen sein, dass der für die Sterilität der Vorformlinge indikative Parameter eine Herstellungstemperatur der Vorformlinge und/oder eine Ausgabetemperatur der Vorformlinge aus der Herstellungsmaschine umfasst. In der Herstellungsmaschine wirkt die verwendete Temperatur zum Herstellen der Vorformlinge üblicherweise sterilisierend. Dies wird jedoch erst ab Überschreiten einer bestimmten Temperatur sichergestellt. Wird der Herstellungsprozess bei einer zu niedrigen Temperatur durchgeführt oder verlassen die Vorformlinge die Herstellungsmaschine mit zu niedriger Temperatur, kann dies darauf hindeuten, dass nicht sämtliche gegebenenfalls auf dem Vorformlingsmaterial vorhandene Keime abgetötet wurden. Die Herstellungstemperatur oder die Ausgabetemperatur können daher als zuverlässige Parameter, die indikativ für die Sterilität des Vorformlings sind, genutzt werden, um Sterilisationsvorgänge zu steuern.

In einer Ausführungsform ist vorgesehen, dass die Anlage eine Heizeinrichtung zum Temperieren der Vorformlinge umfasst und wobei die Heizeinrichtung wenigstens einen Mikrowellenstrahler umfasst. Mikrowellenstrahler bestehen üblicherweise aus Materialien, die beständig gegenüber gängigen Sterilisationsmedien wie H₂O₂ oder O₃ sind und können daher vorteilhaft genutzt werden, um die Vorformlinge vor Zuführen an die Blasformmaschine aufzuheizen bzw. mit einem gewünschten Temperaturprofil zu beaufschlagen, wobei gleichzeitig der Wartungsaufwand und/oder Verschleiß minimiert werden. Die Heizeinrichtung kann alternativ oder zusätzlich auch ein Temperieren der Vorformlinge durch eine oder mehrere Laserquellen und/oder durch Beaufschlagen der Vorformlinge mit Infrarotstrahlung (beispielsweise mittels eines oder mehrerer Infrarotstrahler) bewirken.

Die Behälterbehandlungsmaschine kann einen Füller zum Befüllen von Behältern mit einem Produkt und/oder einen Verschließer zum Verschließen befüllter Behälter und/oder eine Etikettiermaschine zum Aufbringen eines Etiketts auf einen Behälter und/oder eine Direktdruckmaschine zum Aufbringen eines Druckbildes auf einen Behälter umfassen. Insbesondere das Befüllen und/oder Verschließen der Behälter sollte bevorzugt unter sterilen Bedingungen erfolgen. Werden die betreffenden Komponenten aus sterilisationsbeständigem Material hergestellt oder umfassen dieses, kann vorteilhaft auch eine Sterilisation der Behälterbehandlungsmaschine erfolgen, ohne dass hierdurch ein unerwünschter Verschleiß oder Wartungsaufwand entsteht.

Die Anlage kann eine Vorformlingkühleinrichtung zum Kühlen von Vorformlingen mit einem sterilen ersten Kühlmedium und/oder einer Behälterkühleinrichtung zum Kühlen von Behältern mit einem sterilen zweiten Kühlmedium umfassen. Mit dieser Vorformlingkühleinrichtung (die beispielsweise sterile Luft oder Stickstoff als Kühlmedium verwenden kann) wird das Einbringen weiterer Kontaminationsrisiken vermieden.

Es kann vorgesehen sein, dass die mit einem Vorformling oder einem Behälter in physischen Kontakt kommende Komponente wenigstens eines von einem Greifer, einer Klammer, einer Blasform, einer Förderschiene, einem Füllorgan, einer Zentriereinrichtung, einem Stand- oder Drehteller, einem Verschlussorgan, einem in dem Reinraum angeordneten Lufttransporteur zum Transportieren von Behältern und/oder Vorformlingen umfasst.

Diese Komponenten werden üblicherweise zum Befördern oder Behandeln von Behältern eingesetzt. Werden diese oder zumindest ihre mit dem Behälter bzw. Vorformling in Kontakt kommende Oberfläche mit einem sterilisationsbeständigen Material versehen, so reduziert sich selbst bei notwendigen Sterilisationen innerhalb des Reinraums der Wartungsaufwand bzw. der Verschleiß.

Erfindungsgemäß ist weiterhin ein Verfahren zum Herstellen und Behandeln von Behältern mit einer Anlage vorgesehen, die Anlage umfassend einen Reinraum, eine Herstellungsmaschine zum Herstellen von Vorformlingen, eine stromab der Herstellungsmaschine angeordnete Blasformmaschine zum Ausformen der Vorformlinge zu Behältern und eine stromab der Blasformmaschine angeordnete Behälterbehandlungsmaschine zum Behandeln der Behälter, wobei die Herstellungsmaschine, die Blasformmaschine und die Behälterbehandlungsmaschine im Reinraum angeordnet sind und wobei jede mit einem Vorformling oder einem Behälter in physischen Kontakt kommende Komponente der Herstellungsmaschine, der Blasformmaschine und der Behälterbehandlungsmaschine aus sterilisationsbeständigem Material besteht oder sterilisationsbeständiges Material umfasst, das Verfahren umfassend ein Herstellen der Vorformlinge mit der Herstellungsmaschine, ein Zuführen der Vorformlinge an die Blasformmaschine und Ausformen der Vorformlinge zu Behältern und ein Zuführen der Behälter an die Behälterbehandlungsmaschine und Behandeln der Behälter, wobei die Vorformlinge und Behälter mit den Komponenten in physischen Kontakt kommen.

Mit diesem Verfahren ist ein zuverlässiger und insbesondere unter hohen Reinheitsbedingungen erfolgender Betrieb der Anlage zum Herstellen und Behandeln von Behältern sichergestellt.

Es kann vorgesehen sein, dass die Anlage eine Reinraumsterilisationseinrichtung umfasst, die zumindest einen Bereich des Reinraums mit einem Sterilisationsmedium beaufschlagen kann, und wobei das Verfahren ein Beaufschlagen des Bereichs mit dem Sterilisationsmedium umfasst; und/oder
dass die Anlage eine Vorformling-Sterilisationseinrichtung umfasst, die Vorformlinge mit einem Sterilisationsmedium beaufschlagen kann, und wobei das Verfahren ein Beaufschlagen von wenigstens einem Vorformling mit dem Sterilisationsmedium umfasst.

Mit diesen Einrichtungen kann, falls erforderlich, eine zusätzliche Sterilisation erfolgen, beispielsweise wenn der Reinraum zu Wartungszwecken geöffnet wurde oder wenn die Vorformlinge nach ihrer Herstellung nicht die erforderlichen Sterilitätsanforderungen erfüllen.

Insbesondere kann in der zweiten Alternative dieser Ausführungsform vorgesehen sein, dass die Anlage eine Steuereinheit umfasst, die ausgebildet ist, die Vorformling-Sterilisationseinrichtung abhängig von einem für eine Sterilität der Vorformlinge indikativen Parameter zum Beaufschlagen der Vorformlinge mit dem Sterilisationsmedium zu steuern, wobei das Verfahren ein Steuern der Vorformling-Sterilisationseinrichtung zum Beaufschlagen der Vorformlinge mit Sterilisationsmedium durch die Steuereinheit umfasst, wenn der indikative Parameter einen Grenzwert überschreitet oder unterschreitet.

Der Grenzwert kann beispielsweise indikativ dafür sein, dass die Sterilität der Vorformlinge gegeben ist, wobei ein Überschreiten oder Unterschreiten (insbesondere nur eines von beidem) dann ein Kontaminationsrisiko der Vorformlinge darstellen kann. Wird selektiv ein Beaufschlagen der Vorformlinge nur in solchen Fällen durchgeführt, in denen ein Kontaminationsrisiko basierend auf dem indikativen Parameter ermittelt wird, spart dies Sterilisationsmedium und reduziert die Belastung der Anlage.

Es kann vorgesehen sein, dass die Anlage eine Anlage nach einer der vorangegangenen Ausführungsformen ist. Hiermit werden die vorstehend beschriebenen Vorteile der Anlage bei der Herstellung und Behandlung von Behältern realisiert.

### Kurze Beschreibung der Figuren

- Fig. 1: zeigt eine schematische Ansicht einer Anlage zum Herstellen und Behandeln von Behältern
- Fig. 2a-g: zeigen beispielhafte Ausführungsformen von Komponenten mit sterilisationsbeständigem Material

### Ausführliche Beschreibung

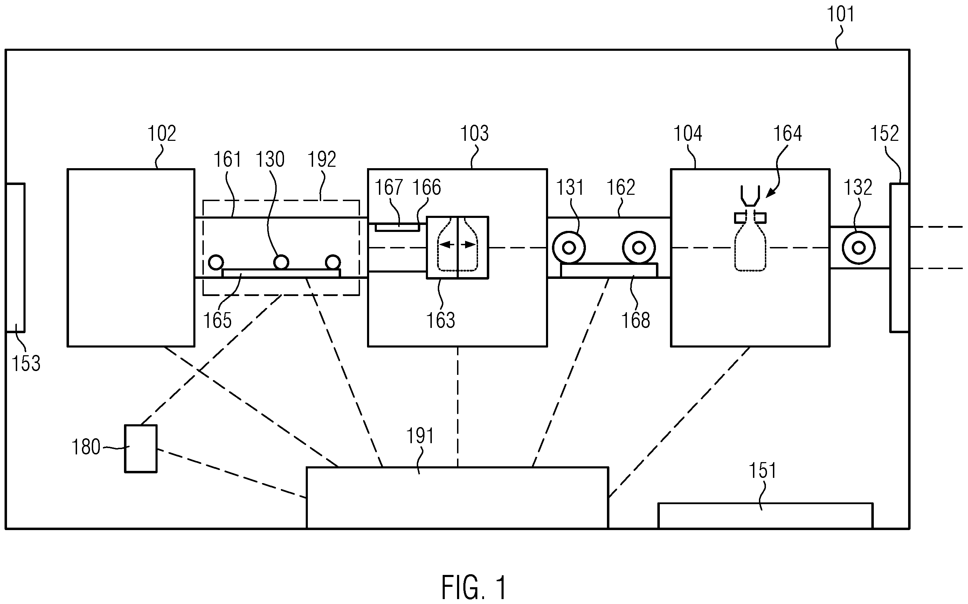

Fig. 1 zeigt eine Ausführungsform einer Anlage 100 zum Herstellen und Behandeln von Behältern 131. Die Anlage 100 umfasst einen Reinraum 101. Dieser Reinraum 101 kann so ausgeführt sein, dass er Reinraumanforderungen, die für die Herstellung und Behandlung der Behälter erforderlich sind, entsprechend den bekannten Reinraumklassen realisieren bzw. erfüllen kann. Dafür können übliche Maßnahmen, wie geeignete Luftzufuhr und/oder -abfuhr sowie Filtersysteme vorgesehen sein. Diese sind aus dem Stand der Technik grundsätzlich bekannt.

Die Anlage 100 umfasst weiterhin eine in dem Reinraum 101 angeordnete Herstellungsmaschine 102, mit der Vorformlinge 130 hergestellt werden können. Die Herstellungsmaschine ist hinsichtlich ihrer Ausgestaltung nicht beschränkt, kann jedoch bevorzugt als Spritzgussmaschine, Compression-Maschine oder Compression-Molding-Maschine ausgeführt sein. Diese Ausgestaltungen sind aus dem Stand der Technik grundsätzlich bekannt. Allgemein ist vorgesehen, dass die Vorformlinge 130 in der Herstellungsmaschine 102 aus einem Vorformlingsausgangsmaterial (beispielsweise PET) mit Hilfe eines Werkzeugs hergestellt werden, wobei ein Erwärmen des Vorformlingsausgangsmaterials soweit erfolgt, dass sich dieses üblicherweise verflüssigt und anschließend dieses verflüssigte Vorformlingsmaterial in geeignete Formen des Werkzeugs eingebracht wird, um aus dem verflüssigten Material durch Abkühlen Vorformlinge herzustellen.

Weiterhin umfasst die Anlage eine Blasformmaschine 103 in dem Reinraum, die mittels einer Blasform 163 aus einem Vorformling 130 einen Behälter 131, wie beispielsweise eine Flasche, herstellen kann. Blasformmaschinen sind grundsätzlich bekannt und bevorzugt handelt es sich hierbei um eine Streckblasmaschine. Diese kann, muss jedoch nicht, als Rundläufermaschine ausgebildet sein, an deren Peripherie um eine Rotationsachse drehbar eine Vielzahl von Blasformen angeordnet ist, die jeweils Vorformlinge aufnehmen und unter Blasdruckeinwirkung sowie optional durch Verstrecken mit einer Reckstange zu einem Behälter ausformen können.

Stromab der Blasformmaschine 103 ist im Reinraum 101 eine weitere Behälterbehandlungsmaschine 104 angeordnet, die die hergestellten Behälter 131 behandeln kann. Bevorzugt umfasst die Behälterbehandlungsmaschine 104 einen Füller zum Befüllen der Behälter 131 mit einem Produkt (etwa einem Getränk, einem Kosmetikprodukt oder einem medizinischen Erzeugnis). Dies ist hier schematisch mit einem Füllorgan 164 dargestellt, das ein Produkt in den Behälter einfüllt. Die Behälterbehandlungsmaschine 104 kann bevorzugt nicht nur einen Füller, sondern zusätzlich oder alternativ auch einen Verschließer umfassen (hier nicht dargestellt), der den befüllten Behälter 131 verschließt. Damit kann eine Anlage 100 realisiert werden, die ein Herstellen und Befüllen sowie Verschließen der Behälter in dem Reinraum 101 realisiert, so dass sämtliche Verfahrensschritte bis zum Verschließen des Behälters, nachdem ein Kontaminationsrisiko des Produkts nicht mehr besteht, unter Reinraumbedingungen realisiert.

Um die Vorformlinge 130 zu der Blasformmaschine zu transportieren, kann eine Transporteinrichtung 161 vorgesehen sein, die die Vorformlinge aus der Herstellungsmaschine 102 empfangen und der Blasformmaschine 103 zuführen kann. Bei der Transporteinrichtung 161 kann es sich beispielsweise um einen Linearförderer, insbesondere einen Luftförderer mit zugehörigen Luftförderschienen handeln, wobei die Vorformlinge zwischen den Schienen beispielsweise auf ihrem Tragring liegend aufgenommen und durch Lufteinwirkung in Richtung der Blasformmaschine transportiert werden können. Alternativ können hier auch ein oder mehrere Drehsterne vorgesehen sein, die die Vorformlinge mit Klammern oder Greifern aufnehmen und von der Herstellungsmaschine zu der Blasformmaschine transportieren können.

Analog kann sich an die Blasformmaschine 103 eine entsprechend ausgeführte Transporteinrichtung 162 anschließen, die die Behälter 131 der nachgeordneten Behälterbehandlungsmaschine 104 zuführt. An die Behälterbehandlungsmaschine 104 kann sich weiterhin eine Transporteinrichtung anschließen, die die behandelten Behälter 132 beispielsweise aus dem Reinraum über eine Abführöffnung 152 abführen kann und beispielsweise weiteren Behandlungsschritten, wie einer Etikettiermaschine oder einer Direktdruckmaschine oder einer Behälterdekorierungsmaschine mit einer Bedruckung oder andersartigen Beschriftung von Behältern z.B. mit einer Laserbeschriftungseinheit, zuführen kann. Die Abführöffnung 152 kann als eine unter Überdruck stehende Schleuse ausgeführt sein, so dass mit Durchführen der behandelten Behälter 132 durch die Abführöffnung 152 keine oder nur eine sehr geringe Kontamination des Innenraums des Reinraums 101 einhergeht.

Analog kann eine Zuführeinrichtung 153 vorgesehen sein, die ebenfalls als Schleuse ausgeführt sein kann und Vorformlingsausgangsmaterial von außerhalb des Reinraums 101 der Herstellungsmaschine bzw. einem der Herstellungsmaschine zugeordneten Vorlagebehälter für Vorformlingsmaterial zuführen kann.

Weiterhin kann ein Zugang 151 vorgesehen sein, durch die Bedienpersonal den Innenraum des Reinraums 101 betreten kann, um beispielsweise Wartungsarbeiten durchzuführen.

Optional kann eine Vorformlingkühleinrichtung 165 beispielsweise im Bereich der Transporteinrichtung 161 angeordnet sein, mit der die Vorformlinge mit einem bevorzugt sterilen Kühlmedium beaufschlagt werden können (beispielsweise sterile Luft oder Stickstoff). Analog kann alternativ oder zusätzlich eine Behälterkühleinrichtung 168 beispielsweise im Bereich der Transporteinrichtung 162 vorgesehen sein, mit der die in der Blasformmaschine 103 hergestellten Behälter 131 gekühlt werden können, bevor diese der nachgeordneten Behälterbehandlungsmaschine zugeführt werden. Auch diese kann bevorzugt die Behälter 131 mit einem sterilen (zweiten) Kühlmedium (beispielsweise sterile Luft oder Stickstoff) beaufschlagen, wobei es sich dabei um dasselbe Kühlmedium handeln kann, mit dem, sofern vorgesehen, die Vorformlingkühleinrichtung die Vorformlinge beaufschlagt.

Während durch das Vorsehen des Reinraums 101 und den darin angeordneten Maschinen der Anlage eine grundsätzlich sterile Herstellung und Behandlung der Behälter möglich ist, kann es auch zu unbeabsichtigten oder nicht vermeidbaren Kontaminationen kommen. Dies ist z. B. dann der Fall, wenn Bediener den Reinraum betreten müssen, um Wartungsarbeiten durchzuführen (beispielsweise defekte Teile auszutauschen oder eine Sortenumstellung durch Austausch von einzelnen Komponenten durchzuführen). Zusätzlich kann während der Herstellung der Vorformlinge aus Vorformlingsmaterial, das üblicherweise nicht in sterilem Zustand der Anlage zugeführt werden kann, ein Kontaminationsrisiko verbleiben, wenn die bei der Herstellung der Vorformlinge verwendeten Temperaturen in der Herstellungsmaschine unterhalb einer Temperatur liegen, bei der eine Sterilisation zuverlässig erfolgt, oder wenn die Zeit für das Herstellen der Vorformlinge zu kurz ist, um eine möglichst vollständige Sterilisation des Vorformlingsmaterials zu bewirken.

Dies kann weitere Sterilisationen des Reinraums 101 und/oder der Vorformlinge 130 erforderlich machen, um sicherzustellen, dass die hergestellten und behandelten Behälter die Sterilitätsanforderungen erfüllen.

Um dies zu ermöglichen und gleichzeitig den Wartungsaufwand bzw. den Verschleiß der Anlage 100 zu minimieren, ist vorgesehen, dass einige, bevorzugt sämtliche mit den Vorformlingen oder den Behältern in physischen Kontakt kommende Komponenten der Herstellungsmaschine, der Blasformmaschine und der Behälterbehandlungsmaschine aus sterilisationsbeständigem Material bestehen oder sterilisationsbeständiges Material umfassen. Dies betrifft alle Komponenten, die ohne zwischenliegendes Material direkt mit den Vorformlingen oder Behältern in Kontakt treten, da diese gegebenenfalls mitsterilisiert werden müssen, wenn sie mit einem potenziell kontaminierten Vorformling oder Behälter in Kontakt gekommen sind oder wenn ein sonstiger Eintrag von Kontamination in den Reinraum 101 erfolgte.

Bevorzugt ist das sterilisationsbeständige Material zumindest auf einer mit den Vorformlingen und/oder Behältern in Kontakt kommenden Oberfläche der Komponenten angeordnet oder diese Komponenten werden vollständig aus dem sterilisationsbeständigen Material (beispielsweise Edelstahl) gefertigt. Unter sterilisationsbeständigem Material ist hier insbesondere ein Material zu verstehen, das gegenüber den üblicherweise in der Lebensmittelindustrie und/oder der Kosmetikindustrie und/oder der Pharmaindustrie zum Einsatz kommenden Sterilisationsmedien beständig ist, also weder korrodiert, noch auf sonstige Weise degeneriert, insbesondere chemisch mit den jeweiligen Sterilisationsmedien reagiert. Hierzu zählt beispielsweise Edelstahl, das gegenüber gängigen Sterilisationsmedien wie H₂O₂ (Wasserstoffperoxid) und Ozon (Os) beständig ist. Entsprechendes Material kann auch oder alternativ gegenüber Peressigsäure beständig sein. Alternativ oder zusätzlich kann das sterilisationsbeständige Material beständig gegenüber Fungiziden sein.

Unter den Vorformlingen und/oder Behältern in Kontakt kommenden Komponenten sind beispielhaft aber nicht abschließend die in der Fig. 1 gezeigten Transporteinrichtungen oder deren mit den Behältern oder Vorformlingen in Kontakt kommende Komponenten sowie beispielsweise die Blasform 164 und das Füllorgan 164 zu verstehen. Werden diese aus sterilisationsbeständigen Materialien gefertigt oder umfassen dieses zumindest, kann auch eine nachfolgende zusätzliche Sterilisation entweder dieser Komponenten oder der Vorformlinge oder Behälter während des Betriebs ohne Beeinträchtigung dieser Komponenten erfolgen, was deren Einsatz ohne oder mit stark reduziertem Verschleiß ermöglicht.

Während grundsätzlich eine maschinelle Sterilisation der Anlage 100 nicht zwingend vorgesehen sein muss, und diese beispielsweise auch manuell je nach Notwendigkeit durch Bediener mit geeignetem Equipment durchgeführt werden kann, kann vorgesehen sein, dass die Anlage eine Reinraumsterilisationseinrichtung 191 umfasst, die ausgebildet ist, um zumindest einen Bereich des Reinraums (beispielsweise spezifisch die Herstellungsmaschine oder die Blasformmaschine oder die Behälterbehandlungsmaschine) mit einem Sterilisationsmedium zu beaufschlagen. Bevorzugt ist jedoch vorgesehen, dass die Reinraumsterilisationseinrichtung den gesamten Reinraum 101 bzw. dessen Innenraum mit einem Sterilisationsmedium beaufschlagen kann. Bevorzugt handelt es sich dabei um ein gasförmiges Sterilisationsmedium wie Ozon oder Wasserstoffperoxid.

Die Reinraumsterilisationseinrichtung 191 kann beispielsweise genutzt werden, um nach durchgeführten Wartungsarbeiten, während denen beispielsweise Bediener durch den Zugang 151 den Reinraum betreten und damit die Luft innerhalb des Reinraums kontaminiert haben, wieder eine vollständige Sterilisation bzw. Reinraumqualität innerhalb des Reinraums 101 herzustellen. Dabei kann vorgesehen sein, dass eine Steuereinheit 180 (beispielsweise die zentrale Steuereinheit der Anlage 100 oder eine separate Steuereinheit) die Reinraumsterilisationseinrichtung 191 basierend auf einem Signal, dass der Zugang 151 wieder verschlossen ist, steuert, um durch diese Sterilisationsmedium in den zumindest den Bereich des Reinraums 101 auszubringen. Beispielsweise kann die Steuereinheit einen Sterilisationszyklus durch die Reinraumsterilisationseinrichtung 191 realisieren, der ein Sterilisieren des Innenraums des Reinraums 101, in dem die Herstellungsmaschine 102, die Blasformmaschine 103 und die Behälterbehandlungsmaschine 104 angeordnet sind, bewirkt, indem beispielsweise Sterilisationsmedium für mehrere Stunden in einer bestimmten Konzentration innerhalb des Reinraums eingebracht wird. Geeignete, hier nicht dargestellte Sensoren (einer oder mehrere) können dabei die Konzentration des Sterilisationsmediums messen und der Steuereinheit durch geeignete Verbindungen zum Datenaustausch übergeben, so dass diese gegebenenfalls die Menge des durch die Reinraumsterilisationseinrichtung 191 eingebrachten Sterilisationsmediums pro Zeiteinheit erhöht oder reduziert, um eine gewünschte Konzentration des Sterilisationsmediums im Reinraum zu realisieren.

Alternativ oder zusätzlich kann auch eine Vorformlings-Sterilisationseinrichtung 192 vorgesehen sein, die so angeordnet sein kann, dass sie die Vorformlinge mit einem Sterilisationsmedium beaufschlagen kann. Beispielsweise kann die Vorformling-Sterilisationseinrichtung 192 in die Herstellungsmaschine 102 integriert sein und/oder im Bereich der Transporteinrichtung 161 angeordnet sein, so dass sie Sterilisationsmedium (flüssig oder gasförmig) auf die Vorformlinge ausbringen kann. Dies kann besonders vorteilhaft genutzt werden, um eine zusätzliche Sterilisation der Vorformlinge und der mit diesen in Kontakt kommenden Komponenten zu bewirken, wenn beispielsweise während der Herstellung der Vorformlinge ein für die Sterilität der Vorformlinge indikativer Parameter anzeigt, dass ein Kontaminationsrisiko der Vorformlinge und damit auch der mit diesen Vorformlingen in Verbindung kommenden Komponenten besteht.

Als geeigneter indikativer Parameter kommt hierfür beispielsweise eine Herstellungstemperatur der Vorformlinge innerhalb der Herstellungsmaschine 102 und/oder eine Ausgabetemperatur der Vorformlinge, mit der die Vorformlinge die Herstellungsmaschine 102 verlassen (beispielsweise die Temperatur, die sie aufweisen, wenn sie der Transporteinrichtung 161 zugeführt werden) infrage. Geeignete, hier nicht dargestellte Sensoren (einer oder mehrere) können diese Temperatur bestimmen und der Steuereinheit durch geeignete Verbindungen zum Datenaustausch übergeben und/oder die Steuereinheit kann aus den Betriebsparametern, mit denen die Vorformlinge in der Herstellungsmaschine 102 hergestellt werden, die Herstellungstemperatur und/oder die Ausgabetemperatur ableiten.

Grundsätzlich kann die Steuereinheit 180 durch einen Vergleich des indikativen Parameters mit einem Grenzwert ermitteln, ob ein Kontaminationsrisiko besteht. Wird dieser Grenzwert überschritten oder unterschritten (je nachdem, ob das Überschreiten oder Unterschreiten indikativ für ein bestehendes Kontaminationsrisiko der Vorformlinge ist), kann die Steuereinheit dann selektiv die Vorformling-Sterilisationseinrichtung 192 zum Beaufschlagen der Vorformlinge mit einem Sterilisationsmedium steuern. Dabei kann bevorzugt eine dynamische Überwachung des indikativen Parameters erfolgen, so dass das Sterilisationsmedium nur dann auf die Vorformlinge und bevorzugt die mit diesen in Kontakt stehenden Komponenten ausgebracht wird, wenn ein Kontaminationsrisiko festgestellt wird und ansonsten kein Sterilisationsmedium ausgebracht wird. Dies reduziert den Verbrauch an Sterilisationsmedium und gleichzeitig die Belastung der Anlage.

Optional kann weiterhin eine Heizeinrichtung 166 zum Temperieren der Vorformlinge vorgesehen sein, die beispielsweise (wie hier dargestellt) als Teil der Blasformmaschine 103 oder stromauf der Blasformmaschine angeordnet sein kann, um die Vorformlinge zu temperieren, insbesondere mit einem Temperaturprofil zu versehen, bevor diese zu Behältern ausgeformt werden.

Bevorzugt umfasst die Heizeinrichtung 166 einen oder mehrere Mikrowellenstrahler 167, die das Temperieren der Vorformlinge durch Beaufschlagen mit Mikrowellen realisieren können. Mikrowellenstrahler sind üblicherweise aus Materialien gefertigt, die gegenüber gängigen Sterilisationsmedien beständig sind, so dass hiermit ebenfalls der Verschleiß der Anlage durch zusätzliches Beaufschlagen mit Sterilisationsmedien reduziert wird. Alternativ oder zusätzlich sind auch Ausführungsformen der Heizeinrichtung mit einer oder mehrerer Laserquellen zum Temperieren der Vorformlinge und/oder mit einem oder mehreren Infrarotstrahlern zum Temperieren der Vorformlinge mit Infrarotstrahlung möglich. Auch beliebige Kombinationen der vorgenannten Ausführungsformen der Heizeinrichtung sind denkbar.

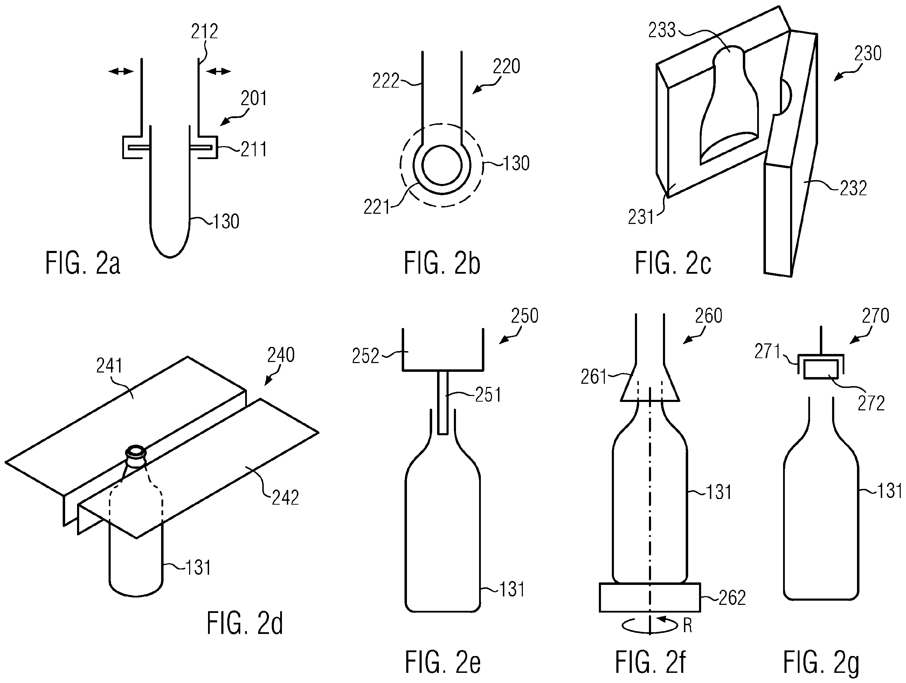

Die Fig. 2a-g zeigen verschiedene Ausführungsformen von Komponenten, die mit sterilisationsbeständigem Material hergestellt sein können oder dieses umfassen können.

In Fig. 2a ist ein Greifer 201 gezeigt, der hier beispielhaft zum Greifen eines Vorformlings 130 an bzw. um seinen Tragring vorgesehen ist. Anstelle des Vorformlings kann der Greifer 201 auch zum Halten bzw. Greifen eines ausgeformten Behälters genutzt werden, wobei analog zum Vorformling auch hier ein Greifen bzw. Halten am Tragring vorgesehen sein kann.

Der Greifer umfasst in der hier gezeigten Ausführungsform einen Greiferbereich 211, der direkt mit dem Vorformling in physischen Kontakt tritt. Zusätzlich umfasst der Greifer einen Verbindungsbereich 212, der beispielsweise mit einer Antriebseinrichtung (hier nicht dargestellt) verbunden sein kann, um den Greifer anzutreiben (etwa zu öffnen oder zu schließen und/oder um den Greifer entlang einer Bewegungsrichtung anzutreiben).

Bevorzugt umfasst zumindest der Teil des Greifers 201, der im Greiferbereich 211 mit dem Vorformling 130 in Kontakt tritt (oder der gesamte Greifbereich 211), das sterilisationsbeständige Material oder ist vollständig daraus gefertigt. Beispielsweise kann vorgesehen sein, dass die Oberfläche des Greiferbereichs 211 aus dem sterilisationsbeständigen Material gebildet ist und ein innerer Bereich des Greiferbereichs 211 von der Oberfläche umschlossen ist und aus anderem Material (beispielsweise Kunststoff) besteht oder dieses umfasst.

Der Verbindungsbereich 212 kann, muss jedoch nicht aus sterilisationsbeständigem Material bestehen. Hiermit können beispielsweise Kosten gespart werden, da die Verwendung von anderen Materialien für Bereiche der Komponente, die nicht mit dem Vorformling oder Behälter in physischen Kontakt treten, weniger häufig einer Sterilisation ausgesetzt sind und daher weniger beständig sein müssen.

Fig. 2b zeigt eine weitere Ausführungsform, in der die Komponente eine Klammer 220 umfasst, die einen Vorformling (oder Behälter) umklammern kann. Das Klammern kann beispielsweise oberhalb des Tragrings (beispielsweise um das Gewinde) oder unterhalb des Tragrings erfolgen oder den Tragring miteinschließen. Die Klammer 220 kann analog zum Greifer 201 einen Klammerbereich 221, der direkt physischen Kontakt mit dem Vorformling bzw. Behälter 130 eingeht, und einen Verbindungsbereich 222 umfassen, der beispielsweise eine Verbindung des Klammerbereichs 221 mit einem Antriebselement zum Betätigen der Klammer zum Freigeben oder Einklemmen eines Vorformlings gewährleistet. Analog zur Fig. 2a kann auch hier vorgesehen sein, dass zumindest der Klammerbereich, optional die gesamte Klammer 220 aus sterilisationsbeständigem Material besteht oder zumindest eine äußere Oberfläche aus sterilisationsbeständigem Material umfasst.

In Fig. 2c umfasst die Komponente eine Blasform 230, mit der aus Vorformlingen in der Blasformmaschine (siehe Fig. 1) Behälter hergestellt werden können. Die hier dargestellte Blasform ist eine zweiteilige Blasform mit zwei Formhälften 231 und 232, deren innere Kontur 233 die Form des herzustellenden Behälters vorgibt. Zumindest die innere Kontur 233 der Blasformhälften kann eine in Richtung des Vorformlings weisende Oberfläche aus sterilisationsbeständigem Material umfassen. Um die Stabilität der Blasform sicherzustellen, kann jedoch vorgesehen sein, dass die Blasformhälften im Wesentlichen einstückig eingebildet sind, insbesondere vollständig aus dem sterilisationsbeständigen Material, wie Edelstahl, bestehen. Anstelle einer zweiteiligen Blasform kann auch eine mehrteilige Blasform (beispielsweise drei Teile mit zwei Blasformhälften und einem Blasformbodenteil) vorgesehen sein, deren gemeinsame innere Kontur die Form des herzustellenden Behälters definiert. Für das Vorsehen des sterilisationsbeständigen Materials gilt hier dasselbe wie für die zweiteilige Blasform beschriebene.

Fig. 2d zeigt eine weitere Ausführungsform einer Komponente 240, die wenigstens eine Förderschiene 241, 242 umfasst. Bevorzugt kann diese Komponente 240 beispielsweise als Luftförderer oder als Teil davon ausgestaltet sein, wobei eine Ausbringeinrichtung zum Ausbringen von Luft in Richtung der Transportrichtung (siehe Pfeil) der Behälter oder Vorformlinge in dem Luftförderer nicht gezeigt ist. Die Förderschienen können so angeordnet sein, dass ein Behälter oder Vorformling zwischen ihnen transportiert werden kann, wobei der Behälter 131 (oder Vorformling) mit seinem Tragring auf den Förderschienen 241 und 242 aufliegen kann. Die Förderschiene bzw. die Förderschienen 241 und 242 können eine in Richtung des Behälters weisende Oberfläche aus sterilisationsbeständigem Material umfassen oder vollständig aus sterilisationsbeständigem Material (etwa Edelstahl) bestehen.

In Fig. 2e ist eine weitere Ausführungsform einer Komponente 250 als Füllorgan dargestellt. Das Füllorgan 250 umfasst einen Füllstutzen 251, der direkt mit dem Behälter 131 in physischen Kontakt treten kann, wobei dies beim Befüllen nicht zwingend der Fall ist, aber zumindest die Möglichkeit des Inkontakttretens besteht. Weiterhin kann das Füllorgan 250 einen Zuführbereich 252 umfassen, der mit dem Füllorgan 251 beispielsweise über Leitungen verbunden ist, um dem Zuführbereich 251 das in den Behälter 131 einzufüllenden Produkts zuzuführen.

Bevorzugt besteht zumindest der Füllstutzen 251 (oder ein Füllventil) aus sterilisationsbeständigem Material oder umfasst dieses (zumindest seine äußere Oberfläche). Bevorzugt kann auch vorgesehen sein, dass weitere Bestandteile des Füllorgans 250, insbesondere der Zuführbereich 252 und hier insbesondere Leitungen, die Produkt führen, aus sterilisationsbeständigem Material bestehen oder eine entsprechende Oberfläche (die in Richtung des zu führenden Produkts weist) umfassen.

Die Fig. 2f zeigt eine weitere Ausführungsform einer Komponente 260. Diese umfasst eine Zentriereinrichtung 261, die hier als Zentrierglocke ausgeführt ist und den Behälter im Bereich seiner Öffnung einspannen kann. Diese Zentriereinrichtung 261 kann eine mit dem Öffnungsbereich des Behälters 131 (beispielsweise seiner Mündung bis zum Tragring) in Kontakt tretende Oberfläche aus sterilisationsbeständigem Material umfassen oder vollständig aus sterilisationsbeständigem Material bestehen. Dabei kann insbesondere vorgesehen sein, dass die Oberfläche Teil eines den Öffnungsbereich des Behälters von außen umfassenden Zentrierelements ist oder dass die Oberfläche Teil eines in den Öffnungsbereich des Behälters hineinragenden Zetrierelements ist. Im ersten Fall wird der Öffnungsbereich des Behälters von dem Zentrierelement zumindest teilweise eingeschlossen. Im zweiten Fall umgibt der Öffnungsbereich das Zentrierelement zumindest teilweise und die Oberfläche ist die äußere Oberfläche des Zentrierelements. Das Zentrierelement kann eine im wesentlichen konische Form besitzen. In der in Fig. 2f dargestellten Ausführungsform umfasst die Komponente 260 weiterhin einen Standteller 262, auf den der Behälter 131 mit seinem Boden aufgestellt werden kann. Der Standteller kann auch als Drehteller 262 ausgeführt sein, um optional in Verbindung mit der Zentriereinrichtung 261 ein Drehen eines Behälters um die hier dargestellte Rotationsachse R zu ermöglichen.

Diese Ausführungsform ist jedoch nicht zwingend und anstelle einer zweiteiligen Komponente aus Zentriereinrichtung 261 und Stand- bzw. Drehteller 262 kann auch nur eine Zentriereinrichtung oder nur ein Stand- bzw. Drehteller als Komponente 260 vorgesehen sein. Auch der Stand- bzw. Drehteller 262 kann zumindest eine äußere Oberfläche aus sterilisationsbeständigem Material umfassen oder vollständig daraus bestehen. Anstelle von Edelstahl kann auch ein sterilisationsbeständiges flexibles Material, wie beispielsweise PU oder Kunststoffe für die mit dem Behälter 131 in Kontakt tretende Oberfläche genutzt werden, da diese einen bezüglich PET hohen Reibungskoeffizienten aufweisen, so dass ein zuverlässiger Halt des Behälters auf den Stand- bzw. Drehteller und/oder in der Zentriereinrichtung gewährleistet bleibt.

Die Fig. 2g zeigt eine Komponente 270 in Form eines Verschließers. Der Verschließer umfasst ein Verschlusselement 271, das einen Verschluss 272 aufnehmen und auf dem befüllten Behälter 131 fest platzieren kann, um den befüllten Behälter zu verschließen. Das Verschlusselement 271 ist bevorzugt zumindest mit einer Oberfläche aus sterilisationsbeständigem Material ausgestattet, die mit dem Behälter 131 beim Verschließen in Kontakt kommt und/oder die mit dem Verschluss 272 in berührenden Kontakt kommt, um hier Kontaminationsrisiken des befüllten Behälters beim Verschließen zu reduzieren und/oder ein weiteres Sterilisieren zu ermöglichen.

Optional kann auch vorgesehen sein, dass der Verschluss oder die Verschlüsse 272, die beispielsweise in einem Verschlussmagazin (in Fig. 2g nicht dargestellt) vorgehalten werden, mit einem Sterilisationsmedium beaufschlagt werden, um die Sterilität des Verschlusses beim Aufbringen auf den Behälter sicherzustellen.

Sämtliche der beschriebenen Komponenten können einzeln oder in Kombination in der Anlage zum Herstellen und Behandeln von Behältern vorgesehen sein, wobei auch beliebige Kombinationen einer oder mehrerer der beschriebenen Komponenten möglich sind.

## Patentansprüche

1. Anlage zum Herstellen und Behandeln von Behältern, die Anlage umfassend einen Reinraum (101), eine Herstellungsmaschine (102)zum Herstellen von Vorformlingen, eine stromab der Herstellungsmaschine angeordnete Blasformmaschine (103) zum Ausformen der Vorformlinge zu Behältern und eine stromab der Blasformmaschine angeordnete Behälterbehandlungsmaschine (104) zum Behandeln der Behälter, wobei die Herstellungsmaschine, die Blasformmaschine und die Behälterbehandlungsmaschine im Reinraum angeordnet sind und wobei jede mit einem Vorformling oder einem Behälter in physischen Kontakt kommende Komponente der Herstellungsmaschine, der Blasformmaschine und der Behälterbehandlungsmaschine aus sterilisationsbeständigem Material besteht oder sterilisationsbeständiges Material umfasst.

2. Anlage nach Anspruch 1, wobei das sterilisationsbeständige Material beständig gegenüber einer Beaufschlagung mit einem gasförmigen und/oder flüssigen Sterilisationsmedium ist.

3. Anlage nach Anspruch 2, wobei das Sterilisationsmedium H₂O₂ und/oder O₃ und/oder Peressigsäure ist.

4. Anlage nach einem der Ansprüche 1 bis 3, wobei die Anlage eine Reinraumsterilisationseinrichtung umfasst, die zumindest einen Bereich des Reinraums mit einem Sterilisationsmedium beaufschlagen kann.

5. Anlage nach einem der Ansprüche 1 bis 4, wobei die Anlage eine Vorformling-Sterilisationseinrichtung umfasst, die Vorformlinge mit einem Sterilisationsmedium beaufschlagen kann.

6. Anlage nach Anspruch 5, wobei die Anlage eine Steuereinheit umfasst, die ausgebildet ist, die Vorformling-Sterilisationseinrichtung abhängig von einem für eine Sterilität der Vorformlinge indikativen Parameter zum Beaufschlagen der Vorformlinge mit dem Sterilisationsmedium zu steuern.

7. Anlage nach Anspruch 6, wobei der für die Sterilität der Vorformlinge indikative Parameter eine Herstellungstemperatur der Vorformlinge und/oder eine Ausgabetemperatur der Vorformlinge aus der Herstellungsmaschine umfasst.

8. Anlage nach einem der Ansprüche 1 bis 7, wobei die Anlage eine Heizeinrichtung zum Temperieren der Vorformlinge umfasst und wobei die Heizeinrichtung wenigstens einen Mikrowellenstrahler und/oder wenigstens eine Laserquelle und/oder wenigstens einen Infrarotstrahler umfasst.

9. Anlage nach einem der Ansprüche 1 bis 8, wobei die Behälterbehandlungsmaschine einen Füller zum Befüllen von Behältern mit einem Produkt und/oder einen Verschließer zum Verschließen befüllter Behälter und/oder eine Etikettiermaschine zum Aufbringen eines Etiketts auf einen Behälter und/oder eine Direktdruckmaschine zum Aufbringen eines Druckbildes auf einen Behälter umfasst.

10. Anlage nach einem der Ansprüche 1 bis 9, wobei die Anlage eine Vorformlingskühleinrichtung zum Kühlen von Vorformlingen mit einem sterilen ersten Kühlmedium und/oder einer Behälterkühleinrichtung zum Kühlen von Behältern mit einem sterilen zweiten Kühlmedium umfasst.

11. Anlage nach einem der Ansprüche 1 bis 10, wobei die mit einem Vorformling oder einem Behälter in physischen Kontakt kommende Komponente wenigstens eines von einem Greifer, einer Klammer, einem Haltedorn, einer Blasform, eine Blasdüse, einer Förderschiene, einem Füllorgan, einer Zentriereinrichtung, einem Stand- oder Drehteller, einem Verschlussorgan, einem in dem Reinraum angeordneten Lufttransporteur zum Transportieren von Behältern und/oder Vorformlingen umfasst.

12. Verfahren zum Herstellen und Behandeln von Behältern mit einer Anlage, die Anlage umfassend einen Reinraum, eine Herstellungsmaschine zum Herstellen von Vorformlingen, eine stromab der Herstellungsmaschine angeordnete Blasformmaschine zum Ausformen der Vorformlinge zu Behältern und eine stromab der Blasformmaschine angeordnete Behälterbehandlungsmaschine zum Behandeln der Behälter, wobei die Herstellungsmaschine, die Blasformmaschine und die Behälterbehandlungsmaschine im Reinraum angeordnet sind und wobei jede mit einem Vorformling oder einem Behälter in physischen Kontakt kommende Komponente der Herstellungsmaschine, der Blasformmaschine und der Behälterbehandlungsmaschine aus sterilisationsbeständigem Material besteht oder sterilisationsbeständiges Material umfasst, das Verfahren umfassend ein Herstellen der Vorformlinge mit der Herstellungsmaschine, ein Zuführen der Vorformlinge an die Blasformmaschine und Ausformen der Vorformlinge zu Behältern und ein Zuführen der Behälter an die Behälterbehandlungsmaschine und Behandeln der Behälter, wobei die Vorformlinge und Behälter mit den Komponenten in physischen Kontakt kommen.

13. Verfahren nach Anspruch 12, wobei die Anlage eine Reinraumsterilisationseinrichtung umfasst, die zumindest einen Bereich des Reinraums mit einem Sterilisationsmedium beaufschlagen kann, und wobei das Verfahren ein Beaufschlagen des Bereichs mit dem Sterilisationsmedium umfasst; und/oder
wobei die Anlage eine Vorformling-Sterilisationseinrichtung umfasst, die Vorformlinge mit einem Sterilisationsmedium beaufschlagen kann, und wobei das Verfahren ein Beaufschlagen von wenigstens einem Vorformling mit dem Sterilisationsmedium umfasst.

14. Verfahren nach Anspruch 13, zweite Alternative, wobei die Anlage eine Steuereinheit umfasst, die ausgebildet ist, die Vorformling-Sterilisationseinrichtung abhängig von einem für eine Sterilität der Vorformlinge indikativen Parameter zum Beaufschlagen der Vorformlinge mit dem Sterilisationsmedium zu steuern, wobei das Verfahren ein Steuern der Vorformling-Sterilisationseinrichtung zum Beaufschlagen der Vorformlinge mit Sterilisationsmedium durch die Steuereinheit umfasst, wenn der indikative Parameter einen Grenzwert überschreitet oder unterschreitet.

15. Verfahren nach einem der Ansprüche 12 bis 14, wobei die Anlage eine Anlage nach einem der Ansprüche 1 bis 11 ist.
